(19) Europäisches Patentamt
European Patent Office
Office européen des brevets

(11) Publication number: **0 297 833 B1**

(12) **EUROPEAN PATENT SPECIFICATION**

(45) Date of publication of patent specification: **22.03.95**

(51) Int. Cl.$^{6}$: **C07K 14/52**, C12N 15/28, A61K 38/19

(21) Application number: **88305875.2**

(22) Date of filing: **27.06.88**

The file contains technical information submitted after the application was filed and not included in this specification

# (54) Physiologically active lymphotoxin.

(30) Priority: **27.06.87 JP 160115/87**

(43) Date of publication of application:
**04.01.89 Bulletin 89/01**

(45) Publication of the grant of the patent:
**22.03.95 Bulletin 95/12**

(84) Designated Contracting States:
**DE FR GB**

(56) References cited:
**EP-A- 0 164 965**

**J. BIOCHEM., vol. 100, no. 3, September 1986, pages 727-73; Y. KOBAYASHI et al.: "Cloning and expression of human lymphotoxin mRNA derived from a human T-cell-hybridoma"**

**J Biol Chem 260 (1985) 2334-44**

(73) Proprietor: **DENKI KAGAKU KOGYO KABUSHIKI KAISHA**
**4-1, Yuraku-cho 1-chome**
**Chiyoda-ku**
**Tokyo (JP)**

(72) Inventor: **Osawa, Toshiaki**
**1-9-13 Akatsuka Shin-machi**
**Itabashi-ku**
**Tokyo (JP)**
Inventor: **Obinata, Masuo**
**Tohoku Daigaku Shukusha 23**
**1-13-10 Kamisugi**
**Sendai-shi**
**Miyagi (JP)**
Inventor: **Ishii, Yoshiyuki**
**238-41, Sonno-cho**
**Chiba-shi**
**Chiba (JP)**

(74) Representative: **Pearce, Anthony Richmond et al**
**MARKS & CLERK,**
**Alpha Tower,**
**Suffolk Street Oueensway**
**Birmingham B1 1TT (GB)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a novel, physiologically active polypeptide, more particularly a novel polypeptide having lymphotoxin activity, and to an antitumor composition comprising said novel polypeptide as an active ingredient.

## BACKGROUND OF THE INVENTION

Lymphotoxin (hereinafter referred to as LT) is known to be a kind of lymphokine which is specifically or nonspecifically released from lymphocytes and lymphoid cell lines and which has cytotoxic activity. LT is not only cytotoxic to various cancer cells but also capable of intensifying the cytotoxic activity of certain anticancer agents or interferons and, therefore, is expected to be of use in the field of medicine as an antitumor agent (Granger, G. A. et al., 14th International Congress of Chemotherapy, Kyoto, Japan, June 23-28, 1985, Abstracts, page 15; Matsunaga, K. et al., ibid., page 352).

A variety of methods have been known for LT production using human-derived cells. According to one method [Peter, J. B. et al., Journal of Immunology, 111, 770 (1973); Walker, S. M. and Lucas, Z. J., ibid, 109, 1233 (1972)], for instance, tonsil cells or peripheral blood lymphocytes are cultured in the presence of a phytohemagglutinin (hereinafter referred to as PHA) and the product LT is isolated from the culture supernatant. According to another method [Yamamoto, R. S. et al., Journal of Biological Response Modifiers, 3, 76 (1984)], a lymphoid cell line is cultured in the presence of phorbol myristate acetate (hereinafter referred to as PMA) and the product LT is isolated from the culture supernatant. According to a further method [Asada, M. et al., Cellular Immunology, 77, 150 (1983)], human T cell hybridomas are cultured in the presence of PMA and/or concanavalin A (hereinafter referred to as Con A).

These known methods, however, have problems in that they can produce LT only in small quantities and require expensive nutrient sources (for example, fetal calf serum) for use in culture media. Therefore, economical production of LT of high purity is very difficult by the known methods.

As an alternative method which might produce LT in large quantities, a method has been suggested which would comprise inserting a gene corresponding to LT into a vector by using the so-called genetic engineering techniques and allowing said gene to be replicated, transcribed and translated in bacteria, fungi, yeasts or animal cells for production of LT in them. Accordingly, isolation of a gene corresponding to LT has been waited for.

The present inventors previously obtained an LT-producing human T cell hybridoma clone, namely the clone A-C5-8, by performing cell fusion by the emetine actinomycin D method [Asada, M. et al., Cellular Immunology, 77, 150 (1983)]. However, when the clone A-C5-8 was cultured in the presence of Con A and/or PMA under optimal LT production conditions (incubation period of 30 hours or longer), no messenger RNA (hereinafter referred to as mRNA) corresponding to LT could be obtained and, therefore, no gene corresponding to LT could be obtained, either.

Therefore, the present inventors made intensive investigations to find out conditions under which an mRNA corresponding to LT could be obtained and, as a result, found that when the clone A-C5-8 is cultured in the presence of PMA and/or Con A for a period not longer than 24 hours (preferably not longer than 4 hours), an mRNA corresponding to the LT polypeptide formed in the cells can be obtained. They then cloned a novel gene coding for the LT polypeptide by applying genetic engineering techniques to said mRNA (Japanese Patent Application (OPI) No. 151182/87 corresponding to EP-A-0230781; the term "OPI" used herein means an unexamined published application.).

The present inventors further conducted intensive investigations and succeeded in producing the LT polypeptide in microorganisms transformed with a phenotypic expression vector with the cloned gene (cDNA) coding for the LT polypeptide being inserted therein. They further succeeded in recovering the human LT polypeptide substantially free of impurities from the thus-obtained, human LT polypeptide-containing cultivation product. They confirmed that this human LT polypeptide can serve as an excellent therapeutic agent for malignant tumors. They have thus accomplished the present invention.

Gray, P. W. et al. [Nature, 312, 721 (1984)] reported a method of producing LT using the recombinant DNA technology. Thus, they cultured nonadherent human peripheral blood lymphocytes in the presence of PMA, staphylococcal enterotoxin B and thymosin $\alpha_1$ for 48 hours, isolated an mRNA from the cells, prepared a cDNA based on said mRNA, ligated the cDNA, after restriction enzyme cleavage, with a synthetic oligonucleotide, and caused LT expression in Escherichia coli. On the basis of the data mentioned in this report, the LT they obtained is estimated to be composed of 171 or 172 amino acid residues and have a molecular weight of 18,600.

The mRNA obtained by the present inventors differs from the LT of Gray et al. in that it is of the human T cell hybridoma origin, that the cDNA derived from the mRNA has a different nucleotide sequence and that the LT polypeptide according to the invention is composed of 151 amino acid residues and has a different molecular weight and a different amino acid sequence.

## SUMMARY OF THE INVENTION

The present invention thus provides a novel polypeptide having LT activity.

More particularly, the invention is concerned with a human LT polypeptide substantially free of impurities as obtained by cleaving the cDNA (Table 1) coding for the LT polypeptide obtained by the procedure described in Japanese Patent Application (OPI) No. 151182/87 with a restriction enzyme, ligating the cleaved cDNA with a phenotypic expression vector having a translation initiation site incorporated therein and cleaved beforehand with the same restriction enzyme, introducing the resulting phenotypic expression vector into an appropriate host, for example *Escherichia coli*, cultivating the thus-obtained transformant, extracting and purifying from the culture thus obtained.

Table 1

```
1                   20                 40                  60
CGGGCTCCTGCACCTGCTGCCTGGATCCCCGGCCTGCCTGGGCCTGGGCCTTGGTTCTCCCC
                80                  100                 120
ATG ACA CCA CCT GAA CGT CTC TTC CTC CCA AGG GTG TGT GGC ACC ACC CTA CAC CTC CTC
                140                 160                 180
CTT CTG GGG CTG CTG CTG GTT CTG CTG CCT GGG GCC CAG GGG CTC CCT GGT GTT GGC CTC
                200                 220                 240
ACA CCT TCA GCT GCC CAG ACT GCC CGT CAG CAC CCC AAG ATG CAT CTT GCC CAC AGC AAC
                260                 280                 300
CTC AAA CCT GCT GCT CAC CTC ATT GGA GAC CCC AGC AAG CAG AAC TCA CTG CTC TGG AGA
                320                 340                 360
GCA AAC ACG GAC CGT GCC TTC CTC CAG GAT GGT TTC TCC TTG AGC AAC AAT TCT CTC CTG
                380                 400                 420
GTC CCC ACC AGT GGC ATC TAC TTC GTC TAC TCC CAG GTG GTC TTC TCT GGG AAA GCC TAC
                440                 460                 480
TCT CCC AAG GCC ACC TCC TCC CCA CTC TAC CTG GCC CAT GAG GTC CAG CTC TTC TCC TCC
                500                 520                 540
CAG TAC CCC TTC CAT GTG CCT CTC CTC AGC TCC CAG AAG ATG GTG TAT CCA GGG CTG CAG
                560                 580                 600
GAA CCC TGG CTG CAC TCG ATG TAC CAC GGG GCT GCG TTC CAG CTC ACC CAG GGA GAC CAG
                620                 640                 660
CTA TCC ACC CAC ACA GAT GGC ATC CCC CAC CTA GTC CTC AGC CCT AGT ACT GTC TTC TTT
GGA
GCA GCC TTC GCT CTG TAG AACTTGGAAAAATCCAGAAA GAAAAAATAATTGATTTCAAGACCTTCTCCCCAT
                680          700                    720
    740           760           780          800
TCTGCCTCCATTCTGACCATTTCAGGGGTCGTCACCACCTCTCCTTTGGCCATTCCAACAGCTCAAGTCTTCCCTGATC
      820           840           860           880
AAGTCACCGGAGCTTTCAAAGAAGGAATTCTAGGCATCCCAGGGGACCCACACCTCCCTGAACCATCCCTGATGTCTGTC
      900           920           940           960
TGGCTGAGGATTTCAAGCCTGCCTAGGAATTCCCAGCCCAAAGCTGTTGGTCTTGTCCACCAGCTAGGTGGGGCCTAGA
      980           1000          1020          1040
TCCACACACAGAGGAAGAGCAGGCACATGGAGGAGCTTGGGGGATGACTAGAGGCAGGGAGGGGACTATTTATGAAGGC
      1060          1080          1100          1120
AAAAAAATTAAATTATTTATTTATGGAGGATGGAGAGAGGGAATAATTAGAAGAACATCCAAGGAGAAACAGAGACAGGC
      1140          1160          1180          1200
CCAAGAGATGAAGAGTGAGAGGGCATGCGCACAAGGCTGACCAAGAGAGAAAGAAGTAGGCATGAGGGATCACAGGGCC
      1220          1240          1260          1280
CCAGAAGGCAGGGAAAGGCTCTGAAAGCCAGCTGCCGACCAGAGCCCCAAACGGAGGCATCTGCATCCTCGATGAAGCC
      1300    1310
CAATAAACCTCTTTTCTCCCCCT
```

Thus, according to one aspect of the present invention, there is provided a homogeneous, physiologically active recombinant human lymphotoxin polypeptide which (a) is substantially free of impurities, (b) has an N-terminal amino acid sequence of Met-His-Leu-Ala-Ser-Asn-Leu-Lys-Pro-Ala-Ala-His-Leu-Ile-Gly-Asp-Pro-Ser-Lys-Gly-Asn-, and (c) has the following amino acid sequence:

```
Met His Leu Ala Ser Asn Leu Lys Pro Ala
1                                   10

Ala His Leu Ile Gly Asp Pro Ser Lys Gly
                                    20

Asn Ser Leu Leu Trp Arg Ala Asn Thr Asp
                                    30

Arg Ala Phe Leu Gly Asn Gly Phe Ser Leu
                                    40

Ser Asn Asn Ser Leu Leu Val Pro Thr Ser
                                    50

Gly Ile Tyr Phe Val Tyr Ser Gly Val Val
                                    60

Phe Ser Gly Lys Ala Tyr Ser Pro Lys Ala
                                    70

Thr Ser Ser Pro Leu Tyr Leu Ala His Glu
                                    80

Val Gly Leu Phe Ser Ser Gly Tyr Pro Phe
                                    90

His Val Pro Leu Leu Ser Ser Gly Lys Met
                                    100

Val Tyr Pro Gly Leu Gln Glu Pro Trp Leu
                                    110

His Ser Met Tyr His Gly Ala Ala Phe Gly
                                    120

Leu Thr Gln Gly Asn Gln Leu Ser Thr His
                                    130

Thr Asn Gly Ile Pro His Leu Val Leu Ser
                                    140

Pro Ser Thr Val Phe Phe Gly Ala Phe Ala
                                    150

Leu.
```

Hereinafter, the above sequence will be referred to as formula (1).

The present invention also resides in DNA coding for a physiologically active peptide having the amino acid sequence of formula (1), and in an antitumor composition which comprises, as an active ingredient, a tumor treating effective amount of a polypeptide having the amino acid sequence of formula (1).

The symbols used in the above formula (1) (polypeptide) and in Table 1 (nucleotide sequence) are defined as follows:

| | | | |
|---|---|---|---|
| ALA | Alanine, | ARG | Arginine, |
| ASN | Asparagine, | ASP | Aspartic acid, |
| CYS | Cysteine, | GLN | Glutamine, |
| GLU | Glutamic acid, | GLY | Glycine, |
| HIS | Histidine, | ILE | Isoleucine, |
| LEU | Leucine, | LYS | Lysine, |
| MET | Methionine, | PHE | Phenylalanine, |
| PRO | Proline, | SER | Serine, |
| THR | Threonine, | TRP | Tryptophan, |
| TYR | Tyrosine, | VAL | Valine, |
| A | Adenine, | C | Cytosine, |
| G | Guanine, | T | Thymine. |

## BRIEF DESCRIPTION OF THE DRAWINGS

Fig. 1 is a schematic representation of the restriction enzyme map obtained in the working example of the present invention.

Fig. 2 is a schematic representation of the restriction enzyme cleavage sites used for the determination of the nucleotide used in the working example of the present invention as well as the directions and ranges for the determination of the nucleotide sequence.

In these figures, B stands for BamHI, E for EcoRI, and P for PstI.

Fig. 3 shows the construction scheme for an expression vector, pDK101, which contains a gene coding for the amino acid sequence of the LT polypeptide of Example 5, where the arrow indicates the direction of the action of the promoter.

Fig. 4 shows the construction scheme for an expression vector, pDK20, which contains a gene coding for the amino acid sequence of the LT polypeptide described in Japanese Patent Application (OPI) No. 8399/88 (corresponding to EP-A-0230781) in Example 10 thereof. The arrow indicates the direction of the action of the promoter.

## DETAILED DESCRIPTION OF THE INVENTION

As far as the amino acid sequences of polypeptides having LT activity are concerned, a report of Aggarwal, B.B., et al [Journal of Biological Chemistry, 260, 2334 (1985)] described that two LTs derived from the human lymphoblast cell line RPMI 1788 have molecular weights of 20,000 and 25,000, respectively. The 6th amino acid residue (ASN) in the amino acid sequence [formula (1)] and the N-terminal amino acid composition of the LT polypeptide according to the invention differ from those in said two LTs. Thus, in the above-mentioned LT having a molecular weight of 20,000, the terminal amino acid HIS is the counterpart to the N-terminal amino acid sequence

**1 4**
**MET-HIS-LEU-ALA**

in formula (1). In the LT having a molecular weight of 25,000, the counterpart to the N-terminal amino acid sequence

**1 4**
**MET-HIS-LEU-ALA**

in formula is LEU-PRO-GLY-VAL-GLY-LEU-THR-PRO-SER-ALA-ALA-GLN-THR-ALA-ARG-GLN-HIS-PRO-LYS-MET-HIS-LEU-ALA-HIS. The three LT polypeptides thus differ from one another on the molecular level.

When a gene is expressed in Escherichia coli, extra methionine residue addition to the N terminus generally occurs, and this may cause adverse effects, such as antigenicity, in applying the expression product as a drug. In accordance with the invention, the 222nd to 224th nucleotides (ATG) shown in Table 1 are used as the initiation codon, so that such extra methionine residue addition can be avoided.

For the wide and safe use of the LT polypeptide as a drug, it is important to isolate the expression product LT polypeptide as a single-component substance.

The lymphotoxins so far known, for example the LT polypeptide described in Japanese Patent Application (OPI) No. 8398/88 (corresponding to EP-A-0230781), are disadvantageous in that the N-terminal portion is unstable and readily undergoes cleavage during cultivation, purification and/or storage and, therefore, further purification is required for obtaining an LT polypeptide having the desired amino acid sequence although the cleavage product polypeptide retains LT activity. In other words, it is difficult to obtain them as homogeneous products. For instance, the LT polypeptide described in Japanese Patent Application (OPI) No. 8398/88 and the lymphotoxin reported by Gray et al., which contains 172 amino acid residues, are apt to be converted, during purification, to LT polypeptides in which part of the N-terminal amino acid sequence is missing. Presumable causes for such cleavage in the N-terminal portion are cleavage due to protease secreted intracellularly during cultivation and/or cell harvesting and cleavage due to various factors during purification steps and storage, for example protease, heat, pH, ion intensity, interaction with resin, solvent and mechanical shearing force. The LT polypeptide according to the invention is very stable to the cleavage factors mentioned above and shows no sign of cleavage in the N-terminal portion and, therefore, the LT polypeptide of formula (1) alone can be obtained in high purity.

As a polypeptide having human LT activity, there may be mentioned the LT polypeptide described in Japanese Patent Application (OPI) No. 8399/88. However, this LT polypeptide differs from the LT polypeptide according to the invention on the molecular level since the N-terminal amino acid sequence of the former is

**1**
**MET-HIS-**

**5**
**LEU-ALA-HIS-SER**

and that of the latter is

**1**
**MET-HIS-LEU-**

**5**
**ALA-SER-ASN.**

Furthermore, the quantity of the LT polypeptide obtainable by inserting the gene coding for the LT polypeptide as described in Japanese Patent Application (OPI) No. 8399/88 into an expression vector to be used in the practice of the invention, allowing said gene to be expressed in the same host as used in the practice of the invention to thereby cause accumulation of the LT polypeptide in the host (cells) and extracting the same therefrom is about one third of the LT polypeptide quantity attainable in accordance with the invention. This suggests that the productivity of the LT polypeptide according to the invention in hosts is very high or that its stability in hosts is superior to the LT polypeptide described in Japanese Patent Application (OPI) No. 8399/88.

The LT polypeptide of the invention as defined by formula (1) has been selected on the basis of the above findings.

The present invention will be explained in further detail hereinbelow.

(1) Selection of cells of high LT productivity:

Usable as the LT-producing cells are normal human lymphocytes, human T-lymphoid cell lines such as CCRF-CEM, MOLT-4F and JURKAT and cloned cell lines thereof as well as human B-lymphoid cell lines such as RPMI-1788. In particular, LT-producing human T-cell hybridomas obtained by cell fusion of normal human T-lymphocytes with an established human T-lymphoid cell line are preferably used since they have high LT productivity and subcultivation of the cells is possible. The LT-producing human T-cell hybridomas are higher in LT productivity than the parent human T-lymphoid cell line and, therefore, larger amounts of mRNA can be extracted and isolated from the cells.

The LT activity measurement was performed by the method of Kobayashi, Y. et al. [Journal of Immunology, 122, 791 (1979)]. Thus, the cytotoxic activity of a sample, namely the culture supernatant or a cell extract, against mouse L•P3 cells (sub-line of L cells) was used as an index. One unit/ml (u/ml) corresponds to that concentration of LT which produces cytopathic effect in 50% of the target cells.

The LT-producing human T-cell hybridomas can be produced by a known method (Japanese Patent Application (OPI) No. 72520/83).

Thus, for instance, human T-lymphoid tumor cells are treated with a protein synthesis inhibitor or with a combination of said inhibitor and an RNA synthesis inhibitor. Separately, human T-lymphocytes are stimulated with a mitogen or an antigen. Both are then hybridized in the presence of a hybridization accelerator (e.g. polyethylene glycol), and the resultant hybrid cells (human T-cell hybridomas) are isolated. The thus-obtained human T-cell hybridomas are incubated in a culture medium (for example, a culture medium prepared by adding 10% of fetal calf serum, $5 \times 10^{-5}$ M of 2-mercaptoethanol and 2 mM of glutamine to the basal medium RPMI-1640, which culture medium is hereinafter referred to as RPMI medium) at 37°C under an atmosphere of $CO_2$ (5%)-air (95%) and the above-mentioned LT-producing human T-cell hybridomas are screened.

(2) Incubation of cells:

For obtaining mRNA from the LT-producing human T-cell hybridomas, at least $10^9$ or more cells are required, and these cells are generally obtained by incubation of said hybridomas. For instance, the cells are put in a nutrient medium in an amount of $10^5$ to $10^7$ cells/ml and incubated in a laboratory dish or in a rotatory flask incubator for tissue culture (spinner flask) under an atmosphere of $CO_2$ (5%)-air (95%) at 37°C.

The incubation period is appropriately 1 to 5 days although it may vary depending on the composition of the medium and/or the initical cell concentration. After incubation, cells are harvested by centrifugation.

The nutrient medium may be a basal medium supplemented with one or more ingredients selected from among saccharides, amino acids, vitamins, hormones, proteins, antibiotics, growth factors, inorganic salts and the like or a basal medium supplemented with an animal serum.

Usable as the basal medium are commercially avialable RPMI-1640 medium, MEM medium, Dulbecco's modified MEM medium, etc.

The animal serum, for example fetal calf serum, neonatal calf serum, equine serum or human serum, can be added to the basal medium in an amount of 1 to 20% of the medium.

Alternatively, the cells may be proliferated in nude mice, hamsters and other warm-blooded animals than humans.

(3) Amplification of mRNA:

The LT-producing human T-cell hybridomas prepared in step (2) are put in a nutrient medium in an amount of $10^6$ to $10^7$ cells/ml, PMA and/or Con A is then added, and incubation is performed. In this manner, cells containing the mRNA corresponding to LT in large amounts can be obtained. The optimal concentration of PMA is generally within the range of 20 to 200 ng/ml and that of Con A within the range of 5 to 50 $\mu$g/ml.

The incubation period should preferably be not longer than 24 hours, more preferably not longer than 8 hours. This is because the content of the mRNA corresponding to the lymphotoxin activity in cultured cells decreases with the lapse of time. If the incubation period exceeds 24 hours, in particular if an incubation period of 24 to 72 hours, which is known to be optimal for the recovery of LT from the culture supernatant in the case of LT-producing human T cell hybridomas in general, is employed, the intracellular amount of the mRNA corresponding to LT becomes very small and it is difficult to recover said mRNA.

(4) Extraction of total RNA from cells:

The extraction of the total cellular RNA from the cells obtained in step (3) can be performed by a known method, for example by the guanidine hydrochloride method [Deeley, R. G. et al., Journal of Biological Chemistry, 252, 8310 (1977)].

Thus, for instance, the cells ($10^9$ or more cells) obtained in step (3) are suspended in a homogenate buffer (containing 8 M of guanidine hydrochloride, 5 mM of dithiothreitol and 20 mM of sodium acetate; adjusted to pH 7 with NaOH) and disrupted by means of a homogenizer or the like. A whole nucleic acid fraction is extracted from the resulting homogenate by ethanol precipitation and phenol extraction and then the total cellular RNA is recovered from the extract by precipitation with lithium chloride. During the extraction procedure, it is recommended that the apparatus and attachments should be dry-heated or treated with diethyl pyrocarbonate and then sterilized in an autoclave and that the operators should wear vinyl plastic gloves on their hands, in order to prevent decomposition of the RNA by the action of RNase.

(5) Isolation of mRNA from total RNA:

The desired mRNA can be isolated from the total cellular RNA by means of conventional methods including sucrose density gradient centrifugation, gel filtration, electrophoresis, membrane filtration, oligo-dT column chromatography, and combinations of these.

For confirming the mRNA thus obtained to be the desired LT-encoding mRNA, it is sufficient to cause the mRNA to be translated into a protein and check the biological activity of said protein. For instance, the mRNA is injected into or added to an appropriate protein synthesis system, such as oocytes of Xenopus laevis, a reticulocyte lysate or wheat germs, for translation therein into a protein, and the resulting protein is checked as to whether it is cytotoxic to mouse L•P3 cells. More precisely, the method comprising the use of Xenopus laevis oocytes is carried out, for example, as follows:

The mRNA is injected into each oocyte in an amount of about 50 to 100 ng by the micro-injection method, and 20 oocytes thus treated are incubated in 200 $\mu$l of a modified Barth's salt solution (containing, per liter thereof, 0.13 g of NaCl, 0.075 g of KCl, 0.2 g of $NaHCO_3$, 0.2 g of $MgSO_4 \cdot 7H_2O$, 0.08 g of $Ca(NO_3)_2 \cdot 4H_2O$, 0.09 g of $CaCl_2 \cdot 6H_2O$, 2.38 g of HEPES, 100 mg of streptomycin and 100,000 units of penicillin G; pH 7.4; hereinafter referred to as MBS) at 23°C for 48 hours. The culture supernatant thus obtained is used as a sample and assayed for LT activity with the cytopathic activity against L•P3 cells as an index.

The LT-encoding mRNA of the present invention has the following characteristics:

(i) It has the sedimentation coefficient of 12.6S to 14.6S;
(ii) It has a polyadenylic acid structure at the 3'-terminus;
(iii) It codes for an LT polypeptide.

(6) Cloning of lymphotoxin cDNA:

The mRNA obtained in step (5) is used as a template and oligo(dT) as a primer, and a single-stranded cDNA which is complementary to the mRNA is synthesized with a reverse transcriptase (e.g. avian myeloblastosis virus-derived reverse transcriptase) in the presence of dATP, dGTP, dCTP and dTTP. The template mRNA is then denatured by heat treatment. Then, this single-stranded cDNA is used as a template and a double-stranded DNA is synthesized by using DNA polymerase I (Klenow fragment) derived from Escherichia coli. This double-stranded DNA is isolated from the denatured mRNA and protein by alkali treatment and phenol extraction. This double-stranded DNA is allowed to undergo the action of a reverse transcriptase for conversion to a more complete double-stranded DNA. The thus-obtained DNA has a hairpin loop structure, which is cleaved with $S_1$ nuclease (Aspergillus oryzae-derived $S_1$ nuclease). The thus-obtained DNA having a complete double-strand structure is inserted, for example, into the plasmid pBR322 at the PstI (restriction enzyme) cleavage site in a conventional manner, for example by the poly(dG)-poly-(dC) or poly(dA)-poly(dT) homopolymer extension method [Noda, M. et al., Nature, 295, 202 (1982); Maniatis, T. et al., "Molecular Cloning (A Laboratory Manual)", 217 (1982), Cold Spring Harbor Laboratory, New York]. The resulting recombinant plasmid is introduced into a host such as Escherichia coli HB101 for transformation thereof in accordance with the method of Perbal, B. ["A Practical Guide to Molecular Cloning", 268 (1984), John Wiley & Sons Inc., Canada], a tetracycline-resistant strain is selected and a DNA library is formed.

The cDNA library is subjected to the colony hybridization test utilizing a synthetic probe [Montgomery, D. L. et al., Cell, 14, 673 (1978); Goeddel, D. V. et al., Nucleic Acids Research, 8, 4057 (1980)], to screen for the desired clone. Thus, two oligonucleotides are chemically synthesized which correspond in a complementary manner to the 18 nucleotides from the 404th to 421st nucleotides and the 18 nucleotides from the 500th to 517th nucleotides in the lymphotoxin gene reported by Gray et al. [Nature, 312, 721 (1984)], respectively, and labeled with $^{32}P$ by phosphate transfer from [$\gamma$-$^{32}P$] ATP to the 5'-terminal hydroxyl group in the presence of a polynucleotide kinase (T4 phage-infected Escherichia coli-derived T4 polynucleotide kinase). The two labeled oligonucleotides are used as probes for selecting, from the above-mentioned cDNA library, a clone capable of strongly binding with both probes. The plasmid DNA is isolated from the thus-obtained clone, converted to the single-stranded form by heating or alkaline denaturation and fixed on a nitrocellulose filter. To this is added a lymphotoxin mRNA-containing mRNA fraction for hybridization, and the bound mRNA is recovered by elution. This is injected into oocytes of Xenopus laevis, and the recovered mRNA is checked as to whether or not it codes for lymphotoxin. (This test is hereinafter referred to as "hybridization-translation test".) The above-mentioned method can give a cloned DNA containing, as an insert, a DNA fragment containing a base sequence complementary to the lymphotoxin mRNA.

Furthermore, this DNA fragment cloned in a pertinent transformant strain may be excised with an appropriate restriction enzyme, labeled with $^{32}P$ and used as a probe for re-screening the above-mentioned cDNA library so that a cDNA fragment of a larger size can be selected.

A restriction enzyme cleavage map of the cloned DNA fragment thus obtained is constructed, the fragment is cloned using M13 phage, the nucleotide sequence of said fragment is analyzed by the dideoxy sequence method of Sanger, E. et al. [Proc. Natl. Acad. Sci. USA, 74, 5463 (1977)], the nucleotide sequence encoding the amino acid sequence of lymphotoxin which has already been determined is traced, and finally, the cDNA containing the nucleotide sequence (i.e. the nucleotide sequence from the 165th to 677th nucleotides in Table I) which corresponds to the complete translation range for lymphotoxin is selected out, whereby the cloned DNA (Table 1) having the nucleotide sequence coding for a polypeptide containing the amino acid sequence of lymphotoxin can be obtained.

A recombinant of the plasmid pBR322 as derived by insertion of the cloned DNA which codes for a polypeptide containing the amino acid sequence of lymphotoxin as shown in Table 1 was named pLT13. A transformant of Escherichia coli HB101 as obtained by transfection with pLT13 has been deposited, under the Budapest Treaty, at the Fermentation Research Institute (Bikoken), Agency of Industrial Science and Technology, Ministry of International Trade and Industry, Japan under the deposit number FERM BP-1226 since November 29, 1986.

(7) Preparation of phenotypic expression vector which contains gene coding for amino acid sequence of LT polypeptide and expression thereof in microorganism:

For causing expression of the gene coding for the amino acid sequence of the LT polypeptide shown in formula (1) in a microorgansim, for example Escherichia coli, it is necessary to insert the LT cDNA gene, together with the initiation codon (ATG) added thereto, downstream from a pertinent promoter such as the tac (trc) promoter or trp promoter. A ribosome binding site is also required 5 to 15 nucleotides upstream from the initiation codon.

Generally, when a mammalian gene is caused to be expressed in a microorganism, for example Escherichia coli, the yield is sometimes low. Presumably, the causes are concerned with, for example (1) the transcription efficiency of the promoter, (2) the nucleotide sequence and secondary structure of the ribosome binding site and the vicinity of ATG, (3) the codon frequencies and (4) degradation of the product. As far as point (1) is concerned, the tac (lac series) promoter or trp promoter is in general use. As regards point (2), an optimum one should be searched for among various ribosome binding sites. As for point (3), mammals differ in codon frequencies from Escherichia coli and, therefore, changes to codons used in Escherichia coli are desirable in some instances. Regarding point (4), introduction into various strains of Escherichia coli and/or incorporation, for more complete control, of a repressor gene into the expression vector is important. It is also important to substitute or delete those nucleotides that correspond to amino acid residues readily cleavable with protease. It is an effective means as well to fuse the nucleotide sequence corresponding to the signal peptide with the LT cDNA so that the expression product can be secreted into the periplasm or culture medium.

In practising the invention, it is recommended that the LT expression vector should be constructed while paying particular attention to points (1), (3) and (4). Thus, trc (a kind of tac promoter) is employed, and the synthetic DNA portion corresponding to the N terminus of LT is modified, for facilitating the subsequent gene manipulation procedure, such that it contains those codons that are best suited for Escherichia coli except for restriction enzyme recognition site introduction. Furthermore, for host screening and for efficient induction, a repressor gene is introduced into the expression vector.

For expression of cDNA encoding LT polypeptide according to the present invention, the known strain of Escherichia coli such as HB101 (Bethesda Research Laboratories), JM105 (Pharmacia), W3110 (ATCC 27325) and Y1089 (ATCC 37196) can be used as a host. The strain W3110 can be preferably used due to high LT-productivity.

The medium for use in cultivating Escherichia coli transformants harboring the LT expression vector constructed in the above-mentioned manner may be a natural medium or a synthetic medium.

As far as the cultivation conditions are concerned, it is necessary to search for those incubation temperature, pH, rate of stirring and rate of aeration which are suited for LT production.

(8) Cultivation of LT polypeptide-producing Escherichia coli and purification of LT polypeptide:

The transformant (LT-producing Escherichia coli) obtained in step (7) is cultivated in an IPTG-containing medium until a sufficient amount of the LT polypeptide is produced. The incubated material is then disintegrated by sonication, by means of an APV Gaulin high-pressure homogenizer, or by a similar means, and the disintegrated matter or the supernatant obtained by centrifugation of the same is subjected to a combination of salting out with ammonium sulfate, ultrafiltration, ion exchange chromatography, gel filtration, heat treatment, reversed phase chromatography, chelate resin chromatography and reversed phase chromatography, whereby the LT polypeptide can be obtained as a substantially pure product.

The LT polypeptide obtained in step (8) was analyzed for amino acid composition and N-terminal amino acid sequence. The data obtained were in agreement with the values estimated from formula (1), as shown in the relevant working example.

The DNA nucleotide sequence coding for the amino acid sequence defined by formula (1) is shown below as nucleotide sequence (1).

Nucleotide sequence (1)

```
                    (5)'-  ATG CAT CTG GCT TCT
AAC CTG AAA CCC GCG GCT CAC CTG ATC GGT
GAC CCG TCT AAA CAG AAC TCT CTG CTG TGG
CGT GCT AAC ACG GAC CGT GCC TTC CTC CAG
GAT GGT TTC TCC TTG AGC AAC AAT TCT CTC
CTG GTC CCC ACC AGT GGC ATC TAC TTC GTC
TAC TCC CAG GTG GTC TTC TCT GGG AAA GCC
TAC TCT CCC AAG GCC ACC TCC TCC CCA CTC
TAC CTG GCC CAT GAG GTC CAG CTC TTC TCC
TCC CAG TAC CCC TTC CAT GTG CCT CTC CTC
```

AGC TCC CAG AAG ATG GTG TAT CCA GGG CTG
CAG GAA CCC TGG CTG CAC TCG ATG TAC CAC
GGG GCT GCG TTC CAG CTC ACC CAG GGA GAC
CAG CTA TCC ACC CAC ACA GAT GGC ATC CCC
CAC CTA GTC CTC AGC CCT AGT ACT GTC TTC
TTT GGA GCC TTC GCT CTG-(3')

(9) Antitumor activity of LT polypeptide:

It has been revealed that the LT polypeptide is cytocidal to animal tumor cells in vitro and in vivo and can be used as an antitumor agent. An antitumor composition according to the present invention comprises a tumor treating effective amount of the LT polypeptide and a pharmaceutically acceptable carrier. The antitumor composition of the present invention can be prepared by mixing the LT polypeptide with a pharmaceutically acceptable stabilizer such as gelatin, serum albumin or maltitol and an appropriate excipient, if desired. The dosage varies dependent on the route of administration, the kind of cancer and the body weight of the patient. The nontoxic amount of the LT polypeptide for a mouse is 1 x $10^3$ to 5 x $10^7$ units/kg/day in the case of intravenous injection (i.v.) or intratumoral injection (i.t.).

The present invention will be explained in greater detail by reference to the following examples, which, however, are not intended to be interpreted as limiting the scope of the present invention.

EXAMPLE 1

Preparation of lymphotoxin-producing human T-cell hybridoma:

Human peripheral blood lymphocytes (hereinafter referred to as PBL; $10^6$ cells/ml) were treated with 20 $\mu$g/ml of concanavalin A (Con A) in RPMI medium for 2 days and, then, the Con A bound to the cells was removed as much as possible by the use of 0.2 M $\alpha$-methyl-D-mannoside.

Separately, human T-lymphoid tumor cells, CCRF-CEM (hereinafter referred to as CEM), which were in a growing stage in RPMI medium were collected by centrifugation. They were suspended in RPMI 1640-10 mM HEPES medium in an amount of 2 x $10^6$ cells/ml. To the suspension were added emetine hydrochloride (Nakarai Chemicals) and actinomycin D (PL Biochemicals) to concentrations of 5 x $10^{-5}$ M and 0.25 $\mu$g/ml, respectively, and the cells were treated at 37°C for 2 hours. Then, the emetine hydrochloride and actinomycin D in the culture were removed by centrifugation.

The PBL cells and CEM cells thus prepared were mixed in a ratio of 10:1 and the mixture was centrifuged. To the cell pellet thus obtained, there were added 0.5 ml of 46% polyethylene glycol (PEG-1540; Wako Pure Chemical Industries) and MEM medium containing 5 $\mu$g/ml of poly-L-arginine and 15% of dimethyl sulfoxide, and the resultant mixture was stirred gently at 37°C for 45 seconds for causing hybridization. Thereafter, 10 ml of MEM medium buffered with 10 ml of 25 mM HEPES (pH 7.2) was gradually added and the resultant mixture was centrifuged.

RPMI medium was added to the cell pellet to make the cell number $10^6$/ml. A 100-$\mu$l portion of the cell-containing medium was mixed with 100 $\mu$l of RPMI medium containing mitomycin C-treated CEM cells (4 x $10^5$ cells/ml) as feeder cells, and the resultant mixture was distributed into wells of a 96-plate culture plate and incubated at 37°C in an atmosphere of $CO_2$ (5%)-air (95%) for about 3 to 4 weeks. After the incubation, the hybridomas that had grown were cloned by the limiting dilution method using the above-mentioned mitomycin C-treated CEM cells as feeder cells. After growing, each clone was assayed for lymphotoxin activity.

Hereinafter, unless otherwise specifically indicated, the incubation was carried out under an atmosphere of $CO_2$ (5%)-air (95%) at 37°C.

The lymphotoxin-producing human T-cell hybridoma clone A-C5-8 to be used in the practice of the present invention, when stimulated with 20 $\mu$g/ml of Con A and 20 ng/ml of PMA at a cell concentration of

2.5 x $10^5$ cells/ml and incubated for 24 hours, showed a lymphotoxin activity of 250 units/ml. When not stimulated, the clone A-C5-8 showed a lymphotoxin activity of 4.0 units/ml.

EXAMPLE 2

Isolation and purification of mRNA fraction from lymphotoxin-producing human T-cell hybridoma (A-C5-8):

(1) Cultivation of lymphotoxin-producing human T-cell hybridoma (A-C5-8):

The clone A-C5-8 was cultivated at a concentration of 5 x $10^6$ to $10^7$ cells/ml for 2, 4, 8, 24 or 48 hours, with PMA and Con A added in final concentrations of 100 ng/ml and 20 μg/ml, respectively.

(2) Preparation of total cellular RNA:

The total cellular RNA of the clone A-C5-8 was extracted essentially by the guanidine hydrochloride method. More detailedly, after each period (mentioned above) of incubation, A-C5-8 cells were collected by centrifugation (1,000 rpm, 5 minutes) and suspended in PBS (containing 5 mM phosphate buffer and 0.15 M of NaCl; pH 7.4), and the suspension was again centrifuged at 1,000 rpm for 5 minutes for washing of the cells.

The cells [in an amount of 4 x $10^8$, 6 x $10^8$, 3.2 x $10^8$, 1.8 x $10^9$, or 2 x $10^9$ cells for the respective incubation period specified in step (1)] were suspended in a homogenate buffer and homogenized therein. To this homogenate were added 0.025 equivalent of 1 M acetic acid and 1.5 volumes of cold ethanol (-20 °C) and, after blending, the mixture was allowed to stand at -20 °C for a period not shorter than 3 hours. The mixture was then centrifuged at -10 °C and at 15,000 rpm (Hitachi RPR 18-2 rotor) for 30 minutes. To the sediment thus obtained was added a washing buffer (prepared by adding EDTA to the homogenate buffer in a concentration of 20 mM; hereinafter referred to as WB) and pipetting was conducted for attaining uniform dissolution. Then, the solution was adjusted to pH 5 by addition of l M acetic acid, 1.5 volumes of cold ethanol was further added, and the mixture was allowed to stand at -20 °C for a period not shorter than 3 hours and, thereafter, centrifuged at -10 °C and 15,000 rpm for 30 minutes. The sediment was dissolved in WB, and the pH adjustment with 1 M acetic acid and precipitation with cold ethanol were repeated three times.

A small amount of 0.1% SDS was added to the ethanol precipitate to make a suspension. To this were added one volume of an extraction buffer (containing 0.5% of SDS, 0.1 M of NaCl, 50 mM of sodium acetate and 5 mM of EDTA disodium; pH 5.2) and two volumes of a water-saturated phenol [containing 0.1% of 8-quinolinol and saturated with 100 mM Tris-HCl buffer (pH 8.0)]-chloroform-isoamyl alcohol mixture (volume ratio = 50:50:1). After 10 minutes of shaking, the whole mixture was centrifuged at 3,000 rpm for 10 minutes, whereby it separated into three layers. The lowest layer was removed, an equal volume of a chloroform-isoamyl alcohol mixture (volume ratio = 50:1) was added to the top and middle layers and the resultant mixture, after shaking for 10 minutes, was centrifuged at 3,000 rpm for 10 minutes. The bottom layer was discarded. This extraction with chloroformisoamyl alcohol was repeated three times. One-tenth volume of 3 M sodium acetate (pH 5.2) and two volumes of cold ethanol were added to the top layer containing DNAs, RNAs, saccharides and so forth, the resultant mixture was allowed to stand at -20 °C for a period not shorter than 3 hours and then centrifuged at 15,000 rpm (RPR 18-2 rotor) for 30 minutes. A small amount of sterile distilled water was added to the sediment. After dissolution, an equal volume of cold 4 M lithium chloride was added, and the mixture was allowed to stand overnight at 0 °C and then centrifuged at 15,000 rpm for 30 minutes. The sediment was washed with a small amount of 2 M lithium chloride, sterile distilled water was added for dissolution of the sediment. After addition of one-tenth volume of 3 M sodium acetate (pH 5.2) followed by addition of 2 volumes of cold ethanol, the resultant mixture was allowed to stand at -20 °C for a period not shorter than 3 hours, followed by 30 minutes of centrifugation at 15,000 rpm. The sediment was dissolved in 0.01 M Tris-HCl buffer (containing 0.5 M of NaCl, 0.1% of SDS and 1 mM of EDTA trisodium; pH 7.5), and the solution containing the total cellular RNA was fed to a column (1 cm in diameter) packed with 3 ml of oligo$(dT)_{12-15}$-cellulose (PL Biochemicals) buffered in advance with the same buffer. After washing with the same buffer until the absorbance at 260 nm reached 0.05 or less, elution was started with 0.01 M Tris-HCl buffer (containing 0.1 M of NaCl, 0.1% of SDS and 1 mM of EDTA trisodium; pH 7.5) and the column was continuously washed until the absorbance at 260 nm reached a level not higher than 0.05. Finally, the polyadenylic acid-bound RNA (mRNA) fraction was eluted from the column with 0.01 M Tris-HCl buffer (containing 0.1% of SDS and 1 mM of EDTA trisodium; pH 7.5) into fraction collectors. Fractions showing a detectable absorbance at 260 nm were pooled. Thus, 58 μg, 130 μg, 180

μg, 120 μg and 258 μg of mRNA were recovered from the A-C5-8 cells incubated for 2, 4, 8, 24 and 48 hours, respectively.

(3) Confirmation of translation, in oocytes, of mRNA corresponding to LT:

Pregnant mare's serum gonadotropin (Peamex injection for veterinary use; Sankyo) was administered to individuals of Xenopus laevis of about 2 years of age (female; weighing not less than 50 g; purchased from Japan Biological Material Center) by intramuscular injection into the thigh in a dose of 200 U/animal. On the next day, the animals were anesthetized by dipping in ice-water and laparotomized, and oocytes were taken out and isolated in MBS. A solution of the mRNA obtained in step (2) in sterile distilled water (1 mg/ml) was injected into the oocytes having a diameter of 1 mm or more in a dose of 50 nl (50 ng) per oocyte by means of a microcapillary and a microinjector (Seimo Scientific Instruments) under a stereoscopic microscope. Twenty oocytes thus treated were incubated in 0.2 ml of MBS at 23°C. In a control run, 20 oocytes given only 50 nl of sterile distilled water were incubated in 0.2 ml of MBS at 23°C.

After 24 or 48 hours of incubation, each culture supernatant was assayed for LT activity As a result, it was found that 48 hours is the optimal incubation period for the oocytes. In addition, it was found that 2 hours is the optimal incubation period for A-C5-8 cells stimulated with Con A and PMA since the LT activity resulting from translation of the mRNA in cells incubated for 2, 4, 8, 24 or 48 hours is 7.8, 4.6, 4.1, 2.7 or 0 units/ml, respectively. No LT activity was detected in control oocytes and thus it was confirmed that the mRNA for LT is translated in the oocytes.

(4) Mass cultivation of A-C5-8 cells and production of mRNA

A-C5-8 cells were grown under the conditions found optimal in step (3) for incubation under stimulation with Con A and PMA, and 3 x $10^9$ cells were harvested. The procedure of step (2) for mRNA isolation and purification was followed and 512 μg of an mRNA fraction was obtained.

(5) Fractionation of mRNA by sucrose density gradient centrifugation and determination of sedimentation coefficient of mRNA corresponding to LT:

The total mRNA (512 μg) was dissolved in 0.01 M Tris-HCl buffer (containing 0.1 M of EDTA disodium and 0.2% of SDS; pH 7.5), the solution was layered on a 5% to 30% sucrose density gradient (5 ml; dissolved in the same buffer), and centrifugation was performed for 16 hours at 15°C and 28,000 rpm by using a Hitachi RPR 55T-208 rotor. The centrifugal tube contents were fractionated in 216-μl portions into 25 vials. One-tenth volume of 3 M sodium acetate and 2 volumes of cold ethanol were added to each fraction, the mixture was allowed to stand overnight at -20°C, and the precipitate mRNA was recovered.

The mRNA thus recovered was dissolved in sterile distilled water (0.5 mg/ml), and 100 nl of the solution was injected into each of 20 Xenopus laevis oocytes in the same manner as in step (3). The oocytes were incubated in 0.2 ml of MBS for 48 hours, and the culture supernatant was assayed for LT activity. As a result, fraction No. 13 was found to have the highest LT activity and, on the basis of a working curve constructed by using standard sedimentation coefficient markers (5S, 18S and 28S), the sedimentation coefficient of the mRNA corresponding to LT was found to be 12.6S to 14.6S.

The purified mRNA obtained in this example was used in the following experiments.

EXAMPLE 3

(1) Synthesis of cDNA:

cDNA synthesis was performed using 5 μg of the purified mRNA in the reverse transcriptase system [$^{32}$P] (New England Nuclear Corp.) partly modified. Thus, reverse transcription was allowed to proceed in a system (100 μl in volume) containing 20 μl of reverse transcriptase reaction buffer, 10 μl of deoxynucleoside triphosphate mixture, 20 units of ribonuclease A inhibitor, 10 μg of oligo(dT)$_{12-18}$ primer, 5 μl of 600 mM β-mercaptoethanol, $^{32}$p-labeled dCTP [specific activity 800 Ci/mmol (100 μCi)], 5 μg of mRNA and 50 units of avian myeloblastosis virus-derived reverse transcriptase at 42°C for 1 hour. The reaction was then terminated by ice cooling. After centrifugation, the hybrid between mRNA and cDNA was heat-treated on a boiling water bath for 3 minutes for separation of mRNA and cDNA from each other, followed by 5 minutes of quenching on an ice bath.

Denatured proteins were caused to form a pellet by centrifugation (12,000 x g, 2 minutes) and the supernatant was again cooled with ice. To the reaction solution (99 µl) were added, with ice cooling, 16.2 µl of sterile distilled water, 46.8 µl of DNA polymerase I reaction buffer, 10.4 µl of deoxynucleoside triphosphate mixture, $^{32}$p-labeled dCTP [800 Ci/mmol (100 µCi)] and 15.6 µl of Escherichia coli-derived DNA polymerase I (8,000 units/ml) to make the total volume 198 µl. The mixture was stirred well, centrifuged, and maintained at 15°C for 20 hours. The reaction was terminated by adding 39.4 µl of 0.2 M EDTA disodium to the reaction solution (197 µl), then 49.25 µl of 1 N NaOH was added, and the mixture was heat-treated at 65°C under alkaline conditions for 1 hour for decomposition of mRNA, then ice-cooled, centrifuged, and neutralized by adding 49.25 µl of 1 M Tris-HCl buffer (pH 8.0) and 49.25 µl of 1 N HCl.

This was extracted with half volume of water-saturated phenol and half volume of chloroform-isoamyl alcohol (50:1). After centrifugation, the upper layer was separated, while the lower layer was re-extracted with an equal volume of 10 mM Tris-HCl buffer (containing 100 mM of NaCl and 1 mM of EDTA disodium; pH 8.0) and the upper layer was separated. Both the upper layers were combined and extracted with an equal volume of chloroform-isoamyl alcohol and, after centrifugation, the lower layer (organic layer) was removed. After four repetitions of this extraction procedure, the aqueous layer was extracted three times with an equal volume of water-saturated ethyl ether. After removal of the organic layer, the aqueous layer was heated on a water bath at 60°C to remove the remaining ethyl ether.

The aqueous layer thus obtained was concentrated with sec-butanol, then $MgCl_2$ (to a final concentration of 0.01 M) and 2 volumes of cold ethanol (-20°C) were added, and the mixture was allowed to stand overnight at -80°C and then subjected to centrifugation (12,000 x g, 10 minutes). The sediment obtained was dried under reduced pressure and dissolved in 50 µl of sterile distilled water. To the solution were added 20 µl of reverse transcriptase reaction buffer, 5 µl of 600 mM β-mercaptoethanol, 10 µl of deoxynucleoside triphosphate mixture, and $^{32}$P-labeled dCTP [800 Ci/mmol (100 µCi)]. After thorough stirring and centrifugation of the mixture, 5 µl of the above-mentioned reverse transcriptase was added, and the reaction was allowed to proceed at 42°C for 1 hour. The reaction was then terminated by ice-cooling. Thus was obtained a cDNA having a hair-pin loop structure.

To the above reaction mixture (99 µl) were added 92.1 µl of sterile distilled water, 23.4 µl of the above-mentioned DNA polymerase I reaction buffer, 55 µl of $S_1$ nuclease reaction buffer and 5.5 µl of Aspergillus oryzae-derived $S_1$ nuclease (50 units/ml), and the reaction was conducted at 37°C for 30 minutes, whereby the hair-pin loop structure was cleaved off and a double-stranded cDNA was formed. To this solution was added 46 µl of 0.1 M Tris-HCl buffer (containing 0.1 M of EDTA disodium; pH 7.5), and the mixture was fed to a Sephacryl S-200 column (1.0 x 25 cm; bed volume 20 ml) and eluted with 10 mM Tris-HCl buffer (containing 0.1 M of NaCl and 1 mM of EDTA disodium; pH 7.5). The eluate was fractionated in 300-µl portions, and a fraction eluted near the void volume was concentrated with sec-butanol and cDNA was recovered therefrom by ethanol precipitation.

(2) Preparation of oligo(dC) tail-added cDNA:

To the double-stranded cDNA obtained as described above was added a reaction buffer having the composition given below, and the reaction was conducted at 37°C for 5 minutes, whereby the oligo(dC) tail was added to the double-stranded cDNA.

The reaction buffer used was 0.2 M potassium cacodylate-25 mM Tris-HCl buffer (pH 6.9) containing 2 mM of DTT, 5 mM of $CoCl_2$, 0.25 mg/ml of BSA, 5 µM of dCTP, $^{3}$H-labeled dCTP [25 Ci/mmole (15 µCi)] and 30 units of terminal deoxynucleotidyl transferase.

The reaction was terminated by ice-cooling, and the reaction mixture was extracted with an equal volume of water-saturated phenol-chloroform-isoamyl alcohol (50:50:1). Extraction was again performed with chloroform-isoamyl alcohol (50:1). Escherichia coli-derived ribosomal RNA (40 µg) and one-fiftieth volume of 5 M NaCl were added to the aqueous layer and, after further addition of 2 volumes of cold ethanol, the mixture was allowed to stand overnight at -80°C. The oligo(dC) tail-added cDNA was recovered by centrifugation and dissolved in sterile distilled water to give a solution having a concentration of 0.5 ng/µl.

(3) Formation of recombinant plasmid:

A 1.375-ng portion of the oligo(dC) tail-added cDNA and 10 ng of oligo$(dG)_{10-20}$ tail-added pBR322 DNA (Amersham) were incubated in 25 µl of an annealing solution (10 mM Tris-HCl buffer containing 100 mM of NaCl and 0.1 mM of EDTA disodium; pH 7.8) at 65°C for 15 minutes, the incubator temperature was then lowered to 45°C, and the mixture was further incubated for 2 hours at that temperature and then cooled with ice for annealing. Thus was obtained a recombinant plasmid-containing solution.

(4) Selection of transformant:

The recombinant plasmid solution obtained as described above was used to transform Escherichia coli HB101. More detailedly, Escherichia coli HB101 was incubated in 10 ml of L-broth containing 0.1% of glucose at 37 °C until the absorbance (at 650 nm) of the culture broth reached 0.05. A 5-ml portion of the culture broth was transferred to 500 ml of L-broth containing 0.1% of glucose and incubation was performed at 25 °C until the absorbance (650 nm) reached 0.3. After cooling with ice for 30 minutes, the culture broth was centrifuged at 4 °C for 5 minutes at 3,000 rpm (Hitachi RPR9-2 rotor) for cell harvesting. The cells collected were dispersed in 250 ml of cold 50 mM $CaCl_2$. The dispersion was ice-cooled for 15 minutes and subjected to centrifugation (RPR9-2 rotor; 4 °C, 5 minutes, 2,500 rpm). The bacterial cells collected were dispersed again in 25 ml of 50 mM $CaCl_2$ containing 2% of glycerol, and the dispersion was distributed in 1-ml portions into vials, frozen with a dry-ice powder and then stored at -80 °C. The stored bacterial dispersion was thawed under ice-cooling, and 0.3 ml of the thus-thawed bacterial dispersion was mixed with 0.15 ml of the above-mentioned recombinant plasmid solution and 0.15 ml of 20 mM $CaCl_2$-60 mM $MnCl_2$-20 mM RbCl mixed solution and the resultant mixture was allowed to stand at 0 °C for 20 minutes and then at room temperature for 10 minutes. Thereafter, 2.4 ml of warmed (37 °C) L-broth containing 0.1% of glucose was added, and the mixture was incubated at 37 °C for 1 hour with shaking. A portion of the thus-incubated broth was taken out, spread over an L-broth-agar plate containing 15 μg/ml of tetracycline in addition to the above-mentioned components and incubated at 37 °C for about 12 hours. Tetracycline-resistant bacterial colonies were selected to prepare a cDNA library.

(5) Hybridization test:

In order to screen out transformants harboring the plasmid containing the LT-encoding cDNA, the above-mentioned cDNA library was subjected to a colony-hybridization test using $^{32}$P-labeled synthetic cDNA probes.

For the formation of the synthetic cDNA probes, two oligonucleotides respectively complementary to the 18 nucleotides from the 404th to 421st nucleotide and the 18 nucleotide from the 500th to 517th nucleotide in the LT gene reported by Gray et al. [Nature, 312, 721 (1984)], namely 5'-GTCTACTCCCAGGTGGTC-3' and 5'-CACATGGAAGGGGTACTG-3', were chemically synthesized, and 16.6 picomoles of each was treated with 5 units of T4 phage-infected Escherichia coli-derived polynucleotide kinase and [γ-$^{32}$P]ATP [5 μCi/pmole (20 μCi)] in 50 mM Tris-HCl buffer (containing 10 mM of $MgCl_2$, 5 mM of DTT, 0.1 mM of spermidine and 0.1 mM of EDTA disodium; pH 7.6] at 37 °C for 30 minutes.

The reaction was terminated by adding an EDTA solution and cooling the mixture with ice to 0 °C. These two $^{32}$P-labeled cDNA probes were used in the hybridization test, and transformants carrying a recombinant plasmid capable of being hybridized with both of the two probes under weak conditions were selected. Six colonies were thus selected out from among about 10,000 colonies.

The recombinant plasmid was recovered from each of the 6 strains thus selected. The plasmid was cleaved with the restriction enzyme HindIII, subjected to agarose gel electrophoresis, transferred to a nitrocellulose filter and then again hybridized with the $^{32}$P-labeled synthetic cDNA porbes under severe conditions. As a result, one clone was selected out.

The thus-selected strain was subjected to a hybridization-translation test, which was conducted by the method described by Maniatis, T. et al. in Molecular Cloning, 329 (1980) (Cold Spring Harbor Laboratory). The plasmid DNA was extracted from this transformant strain and, after heating and denaturation, it was fixed on a nitrocellulose filter. The LT mRNA-containing mRNA fraction obtained in step (4) of Example 2 was added thereto and the hybridization reaction was effected at 50 °C for 3 hours. The bound mRNA was recovered by elution and injected into Xenopus laevis oocytes in the same manner as in step (3) of Example 2, and the recovered mRNA was checked as to whether it was the LT mRNA or not.

As a result, 10 units/ml of LT activity was detected in the case of pLT13, whereas no LT activity was detected in the control case of pBR322.

This cDNA was cleaved with the restriction enzyme HindIII, and the size thereof was measured by agarose gel electrophoresis. As a result, it was confirmed that said cDNA had a cDNA segment of about 1.35 Kbp.

The transformant containing this cDNA (strain number LT13; cloned DNA number: pLT13) was treated for the isolation of the cloned cDNA, and the nucleotide sequence thereof was determined by the method mentioned below.

EXAMPLE 4

Determination of nucleotide sequence of cloned DNA:

(1) Construction of restriction enzyme cleavage map:

The strain LT13 obtained in step (5) of Example 3 was incubated in L-broth containing 0.1% of glucose and 15 μg/ml of tetracycline. From the bacterial cells obtained, the plasmid DNA was recovered, and a restriction enzyme cleavage map thereof was drawn for the restriction enzymes EcoRI, SmaI, BamHI, PstI, SalI and HindIII whose recognition sites can be introduced into M13mp8 and M13mp9, as will be mentioned below. (Cf. Fig. 1.)

(2) Determination of nucleotide sequence of cloned DNA:

For the determination of the nucleotide sequence of the cloned DNA, the cDNA fragment was subcloned in M13mp8 or M13mp9 by the method of Messing et al. [Gene, 19, 269 (1982)] and then processed by the dideoxy chain termination method of Sanger et al. [Proc. Natl. Acad. Sci. USA, 74, 5463 (1977); Journal of Biological Chemistry, 162, 729 (1982)] for primer annealing, complementary chain synthesis using the Klenow fragment of DNA polymerase I [as labeled with $^{32}$P-labeled dCTP [800 Ci/mmol (20 μCi)]], gel electrophoresis, and autoradiography.

Fig. 2 shows the restriction enzyme cleavage sites used for the nucleotide sequence determination, with the direction used for the nucleotide sequence determination and the range of sequencing being indicated by arrows therein. The section designated by a rectangle therein is the part encoding the LT translation range. The nucleotide sequence from the 63rd to 677th nucleotides presumably encodes a polypeptide necessary for the construction of a precursor for LT.

Among these nucleotides, those beginning with the 165th nucleotide are considered to encode LT, and the nucleotide sequence is different from that of LT as reported by Gray, P. W. et al. [Nature, 312, 721 (1984)] in that the 26th amino acid is Asn (AAC) in the former while the amino acid is Thr (ACC) in the latter. The present sequence has the termination codon (TAG) following the C-terminal amino acid (leucine) codon. In addition, the present sequence is further different from the sequence of Gray et al. in parts other than the peptide-encoding part in that the 1st to 4th nucleotides are CGGG in place of GGTC, that the 862nd to 865th nucleotides are ACAC in place of CACA and that the 1306th to 1310th nucleotides are CCCCT in place of TGAAA.

EXAMPLE 5

(1) Cleavage of LT cDNA with restriction enzymes:

The cloned DNA (pLT13) obtained in Example 3 was introduced into Escherichia coli SK383 (a dam$^{-}$ strain; available from Tokyo University) because of the restriction enzyme BclI being sensitive to dam, and the plasmid DNA was prepared.

This pLT13 was digested with BclI and dephosphorylated at the 5' ends with bacterial alkaline phosphatase (BAP; Bethesda Research Laboratories).

The following HindIII linker was prepared:

```
GATCAAGCTT
    TTCGAACTAG
```

The two oligonucleotides were phosphorylated at the 5' end with T4 DNA kinase (Takara Shuzo) and then annealed to give the double-stranded DNA.

The BclI digest of pLT13 and the HindIII linker were ligated together in the presence of T4 DNA ligase and the ligation product was introduced into Escherichia coli HB101. A plasmid, pLT13H, with the HindIII linker incorporated therein was obtained.

pLT13H was digested with RsrII and HindIII and the digest was fractionated in a conventional manner by 4% acrylamide gel electrophoresis, and a fragment of 502 bp was recovered.

(2) Synthesis of oligonucleotides:

Two oligonucleotides respectively having the structures shown below, namely L1 (88mer) and L2 (87mer), were synthesized by the $\beta$-cyanoethylphosphamidide method (Biosearch model 8600). The synthetic oligonucleotides were purified by reversed phase chromatography (C18 column; Waters) in accordance with the Biosearch protocol. After phosphorylation at the 5' end, they were annealed to give a double-stranded DNA.

L1 : 5'-CATGCATCTGGCTTCTAACCTGAAACCCGCGGCT-
CACCTGATCGGTGACCCGTCTAAACAGAACTCTC-
TGCTGTGGCGTGCTAACACG-3'

L2 : 5'-GTCCGTGTTAGCACGCCACAGCAGAGAGTTCTGT-
TTAGACGGGTCACCGATCAGGTGAGCCGCGGGTT-
TCAGGTTAGAAGCCAGATG-3'

(3) Introduction of LT cDNA fragment into expression vector:

The expression vector pKK233-2 (Pharmacia) was digested with NcoI and HindIII, the digest was fractionated in a conventional manner by 0.7% agarose gel electrophoresis, and the vector was recovered. The DNAs prepared in step (1) and step (2) and the above vector were ligated together, the ligation product was introduced into Escherichia coli JM105, and selection was performed in LB medium containing ampicillin. About 2,500 transformants were obtained.

(4) Screening with synthetic probe:

The transformants obtained in step (3) were subjected to the colony hybridization test using, as a probe, the synthetic oligonucleotide L1 prepared in step (2) labeled with [$\gamma$-$^{32}$P] ATP at the 5' end. Among the transformants that had hybridized, 24 clones were selected and digested with NsiI (Bethesda Research Laboratories) and RsrII (New England Biolabs). The digests were subjected to agarose gel electrophoresis, and the desired fragment was searched for and the directionality thereof was determined. A plasmid, pDK100, was obtained as the desired one.

(5) Determination of nucleotide sequence of synthetic DNA:

The plasmid pDK100 obtained in step (4) was digested with NsiI and HindIII, the digest was fractionated by agarose gel electrophoresis, and the LT cDNA fragment was recovered. This fragment was inserted into M13mp19 between the PstI and HindIII sites, and the base sequence of the synthetic DNA portion was determined by the dideoxy method and found to be in agreement with the desired one.

(6) Introduction of repressor gene:

The plasmid pMJR1560 (Amersham) was digested with HindIII, the 5' ends were made blunt with DNA polymerase I (Klenow fragment), the blunt-end digest was further digested with EcoRI, the digest obtained was fractionated by 1% agarose gel electrophoresis, and a 1,261-bp fragment containing the lac repressor (lac $I^q$) gene.

The plasmid pDK100 obtained in step (4) was digested with EcoRI and PvuII, a fragment containing the LT gene was recovered and ligated with the lac repressor gene-containing fragment, and the ligation product was introduced into Escherichia coli HB101. Thus was obtained pDK101. The construction process including the above steps (1) to (6) is shown in Fig. 3.

(7) Introduction into various Escherichia coli strains:

The DNA of the plasmid pDK101 obtained in step (6) was purified and introduced into Escherichia coli JM105, W3110 (ATCC 27,325) and Y1089 (ATCC 37,196).

EXAMPLE 6

The Escherichia coli strains HB101, JM105, W3110 and Y1089 carrying the plasmid pDK101 obtained in step (7) of Example 5 were each cultured in LB-medium (see Note 1 given below) containing 50 µg/ml of ampicillin until the $O.D._{550}$ reached 0.5. Then, isopropyl-β-D-thiogalactoside (IPTG) was added to a final concentration of 1 mM, and incubation was continued for further 4 hours. Cells were harvested, washed, suspended in disintegration buffer [50 mM Tris-HCl (pH 8.0), 10 mM EDTA, 30 mM NaCl, 0.01 mM (p-amidinophenyl)methanesulfonyl fluoride] and disintegrated by the lysozyme/freezing-thawing method, and the supernatant was assayed for LT activity. The activity data thus obtained are shown in Table 2.

Table 2

| Strain [plasmid] | Activity (units/ml) | Specific activity (units/ml $OD_{550}$) |
|---|---|---|
| HB101[pDK101] | 230,000 | $4.4 \times 10^4$ |
| JM105[pDK101] | 200,000 | $5.1 \times 10^4$ |
| W3110[pDK101] | 500,000 | $1 \times 10^5$ |
| Y1089[pDK101] | 210,000 | $4.6 \times 10^4$ |
| Note 1: Composition of LB-medium, per liter: Bacto-Tryptone 10 g, Bacto yeast extract 5 g, NaCl, 10 g; pH 7 to 7.5. | | |

EXAMPLE 7

The Escherichia coli strain W3110[pDK101] obtained in Example 6 was cultivated by the method of Example 6, and 10 liters of a culture was obtained. This was concentrated by ultrafiltration, and cells were disrupted by means of a high-pressure homogenizer (APV Gaulin). Polyethylenimine (final concentration 0.5%) was added to the homogenate for nucleic acid removal and, then, salting out with ammonium sulfate (40% saturation) was performed. The precipitate obtained was dissolved in 5 mM Tris-HCl buffer (pH 8). The solution was heat-treated at 60°C for 30 minutes and applied to a DEAE-Cellulofine AM column (Chisso Corp.; 9 x 50 cm) for adsorption. Elution was carried out on a concentration gradient of 0 to 0.2 M NaCl. An active fraction was applied to a zinc chelate Sepharose column (Pharmacia; 1.6 x 20 cm) for adsorption, followed by elution on a concentration gradient of 0 to 0.2 M imidazole (pH 7.5). An active fraction was subjected to gel filtration using a Sephacryl S-200 column (Pharmacia; 5 x 100 cm), which gave an active fraction (S-200-fractionated fraction). The LT polypeptide in the S-200-fractionated fraction had a specific activity of $7 \times 10^7$ units/mg protein, and the activity recovery from the cell homogenate was 30%. The molecular weight of the LT polypeptide was determined by subjecting the S-200-fractionated fraction to electrophoresis on a 0.1% SDS-containing polyacrylamide gel (SDS-PAGE) [Laemmli, U. K., Nature, 227, 680 (1970)] and found to be about 16,000. Furthermore, any decomposition (decrease in molecular weight) of the LT polypeptide was not observed at all in each purification step (SDS-PAGE analysis).

Table 3

Amino acid composition of LT polypeptide

| Amino acid | Measured value (moles) | Calculated value based on formula (1) (moles) |
|---|---|---|
| ILE | 2.9 | 3 |
| VAL | 9.1 | 9 |
| LEU | 21.5 | 21 |
| PHE | 10.1 | 10 |
| CYS | < 0.1 | 0 |
| MET | 2.9 | 3 |
| ALA | 11.9 | 12 |
| GLY | 9.0 | 9 |
| THR | 7.1 | 7 |
| TRP | 1.8 | 2 |
| SER | 19.7 | 20 |
| TYR | 6.7 | 7 |
| PRO | 11.5 | 11 |
| HIS | 8.5 | 8 |
| GLU } GLN } | 11.8 | 2<br>10 |
| ASP } ASN } | 10.1 | 5<br>5 |
| LYS | 5.0 | 5 |
| ARG | 2.1 | 2 |

EXAMPLE 8

The S-200-fractionated fraction obtained in Example 7 was purified on a C18 reversed-phase chromatography column (Waters; 0.39 x 30 cm) and the C18-fractionated fraction was submitted to analysis for amino acid composition and analysis for N-terminal amino acid sequence.

(1) Amino acid composition:

The C18-fractionated fraction was evaporated to dryness under reduced pressure. On a work station (Waters), the solid obtained was hydrolyzed with hydrochloric acid and the hydrolyzate was reacted with phenyl-isothiocyanate for conversion to PTC-amino acids, and composition analysis was perfomed using an amino acid analyzer (reversed phase partition method; Waters). Quantitative determination of tryptophan and cysteine was carried out by the method of Inglis, A. S. (Methods in Enzymology, vol. 91, page 26, edited by Hirs, C. H. W. et al., Academic Press, 1983). The results obtained are shown in Table 3.

The measured values were in good agreement with the values calculated on the basis of formula (1).

(2) N-Terminal amino acid sequence:

The N-terminal amino acid sequence was determined by Edman's method [Edman, P., Arch. Biochem. Biophys., 22, 475 (1949)] by using a gaseous phase protein sequencer (Applied Biosystems model 477A) and a PTH analyzer (Applied Biosystems model 120A).

The thus-found N-terminal amino acid sequence of the LT polypeptide, namely
MET-HIS-LEU-ALA-SER-ASN-LEU-LYS-PRO-ALA-
ALA-HIS-LEU-ILE-GLY-ASP-PRO-SER-LYS-GLN-ASN-
was in complete agreement with the sequence from the N-terminal amino acid to the 21st amino acid of formula (1).

EXAMPLE 9

Balb/c mice (8 weeks old females; purchased from Nippon Charles River) were inoculated, by subcutaneous injection into the right inguinal region, with Meth A mouse tumor cells at an inoculum size of $1 \times 10^5$ cells per animal (day 0). On day 7, the tumor size was 40 to 60 $mm^2$. An LT preparation prepared by diluting the S-200-fractionated fraction obtained in Example 7 with PBS containing 100 $\mu$g/ml of gelatin was administered to the mice either intravenously (i.v.) or intratumorally (i.t.) for consecutive 5 days beginning with day 7. Complete tumor disappearance was attained on day 25 in the i.v. group and on day 20 in the i.t. group at an LT dose of $1 \times 10^4$ units/mouse/day and of $5 \times 10^2$ units/mouse/day, respectively.

EXAMPLE 10

(1) Construction of expression vector for production of LT described in Japanese Patent Application (OPI) No. 8399/88.

The plasmid pDK101 obtained in step (6) of Example 5 was digested with NsiI and HindIII, the digest was fractionated in a conventional manner by 0.7% agarose gel electrophoresis, and the vector was recovered. Separately, pLT13H obtained in step (1) of Example 5 was digested with NsiI and HindIII, the digest was fractionated in a conventional manner by 1.2% agarose gel electrophoresis, and a fragment containing the LT gene was recovered. The vector and the LT gene-containing fragment were ligated together, the ligation product was introduced into Escherichia coli W3110, and an LT expression vector, pDK20, was obtained. The construction process mentioned above is illustrated in Fig. 4.

(2) Introduction of pDK20 into Escherichia coli W3110, LT production, and LT productivity comparison with W3110[pDK101]:

The DNA of pDK20 obtained in step (1) was purified and introduced into W3110 (transformant strain name: W3110[pDK20 ]). W3110[pDK20] and W3110[pDK101] were each cultured in LB-medium containing 50 $\mu$g/ml of ampicillin until $O.D._{550}$ = 0.5, IPTG was then added to a final concentration of 1 mM, and incubation was conducted for further 4 hours. Cells were collected, washed, suspended in disintegration buffer [50 mM Tris-HCl (pH 8.0), 10 mM EDTA disodium, 30 mM NaCl, 0.01 mM (p-amidinophenyl)-methanesulfonyl fluoride] and disintegrated by the lysozyme/freezing-thawing method, and the supernatant was assayed for LT activity. As a result, as shown in Table 4, it was revealed that the LT polypeptide of the present invention can be produced about three times more abundantly in Escherichia coli as compared with the LT polypeptide described in Japanese Patent Application (OPI) No. 8399/88.

Table 4

| Strain [plasmid] | Activity (units/ml) | Specific activity (units/ml $OD_{550}$) |
|---|---|---|
| W3110[pDK101] | 520,000 | 1.15 x $10^5$ |
| W3110[pDK20] | 170,000 | 0.36 x $10^5$ |

REFERENCE EXAMPLE

The LT polypeptide of formula (2) (shown below) described in Japanese Patent Application (OPI) No. 8399/88, when subjected to the purification steps described in Example 8, partly gave polypeptides lacking in the N-terminal amino acid sequence MET-ASP-PRO-ALA-GLN-THR-ALA-, MET-ASP-PRO-ALA-GLN-THR-ALA-ARG-GLN-HIS-PRO-LYS-MET- or MET-ASP-PRO-ALA-GLN-THR-ALA-ARG-GLN-HIS-PRO-LYS-MET-HIS-.These polypeptides all had LT activity.

Formula (2)

MET ASP PRO ALA GLN THR ALA ARG GLN HIS
PRO LYS MET HIS LEU ALA HIS SER ASN LEU
LYS PRO ALA ALA HIS LEU ILE GLY ASP PRO
SER LYS GLN ASN SER LEU LEU TRP ARG ALA
ASN THR ASP ARG ALA PHE LEU GLN ASP GLY
PHE SER LEU SER ASN ASN SER LEU LEU VAL
PRO THR SER GLY ILE TYR PHE VAL TYR SER
GLN VAL VAL PHE SER GLY LYS ALA TYR SER
PRO LYS ALA THR SER SER PRO LEU TYR LEU
ALA HIS GLU VAL GLN LEU PHE SER SER GLN
TYR PRO PHE HIS VAL PRO LEU LEU SER SER
GLN LYS MET VAL TYR PRO GLY LEU GLN GLU
PRO TRP LEU HIS SER MET TYR HIS GLY ALA
ALA PHE GLN LEU THR GLN GLY ASP GLN LEU
SER THR HIS THR ASP GLY ILE PRO HIS LEU
VAL LEU SER PRO SER THR VAL PHE PHE GLY
ALA PHE ALA LEU

## Claims

1. A homogeneous, physiologically active recombinant human lymphotoxin polypeptide which (a) is substantially free of impurities, (b) has an N-terminal amino acid sequence of Met-His-Leu-Ala-Ser-Asn-Leu-Lys-Pro-Ala-Ala-His-Leu-Ile-Gly-Asp-Pro-Ser-Lys-Gly-Asn-, and (c) has the following amino acid sequence:

Met His Leu Ala Ser Asn Leu Lys Pro Ala
1 10

Ala His Leu Ile Gly Asp Pro Ser Lys Gly
20

Asn Ser Leu Leu Trp Arg Ala Asn Thr Asp
30

Arg Ala Phe Leu Gly Asn Gly Phe Ser Leu
40

Ser Asn Asn Ser Leu Leu Val Pro Thr Ser
50

Gly Ile Tyr Phe Val Tyr Ser Gly Val Val
60

Phe Ser Gly Lys Ala Tyr Ser Pro Lys Ala
70

Thr Ser Ser Pro Leu Tyr Leu Ala His Glu
80

Val Gly Leu Phe Ser Ser Gly Tyr Pro Phe
90

His Val Pro Leu Leu Ser Ser Gly Lys Met
100

Val Tyr Pro Gly Leu Gln Glu Pro Trp Leu
110

His Ser Met Tyr His Gly Ala Ala Phe Gly
120

Leu Thr Gln Gly Asn Gln Leu Ser Thr His
130

Thr Asn Gly Ile Pro His Leu Val Leu Ser
140

Pro Ser Thr Val Phe Phe Gly Ala Phe Ala
150

Leu.

2. DNA coding for a physiologically active polypeptide having the amino acid sequence:

```
Met His Leu Ala Ser Asn Leu Lys Pro Ala
1                                    10
Ala His Leu Ile Gly Asp Pro Ser Lys Gly
                                     20
Asn Ser Leu Leu Trp Arg Ala Asn Thr Asp
                                     30
Arg Ala Phe Leu Gly Asn Gly Phe Ser Leu
                                     40
Ser Asn Asn Ser Leu Leu Val Pro Thr Ser
                                     50
Gly Ile Tyr Phe Val Tyr Ser Gly Val Val
                                     60
Phe Ser Gly Lys Ala Tyr Ser Pro Lys Ala
                                     70
Thr Ser Ser Pro Leu Tyr Leu Ala His Glu
                                     80
Val Gly Leu Phe Ser Ser Gly Tyr Pro Phe
                                     90
His Val Pro Leu Leu Ser Ser Gly Lys Met
                                    100
Val Tyr Pro Gly Leu Gln Glu Pro Trp Leu
                                    110
His Ser Met Tyr His Gly Ala Ala Phe Gly
                                    120
Leu Thr Gln Gly Asn Gln Leu Ser Thr His
                                    130

Thr Asn Gly Ile Pro His Leu Val Leu Ser
                                    140
Pro Ser Thr Val Phe Phe Gly Ala Phe Ala
                                    150
Leu.
```

3. An antitumor composition which comprises, as an active ingredient, a tumor treating effective amount of a polypeptide having the amino acid sequence:

```
Met His Leu Ala Ser Asn Leu Lys Pro Ala
1                                   10

Ala His Leu Ile Gly Asp Pro Ser Lys Gly
                                    20

Asn Ser Leu Leu Trp Arg Ala Asn Thr Asp
                                    30

Arg Ala Phe Leu Gly Asn Gly Phe Ser Leu
                                    40

Ser Asn Asn Ser Leu Leu Val Pro Thr Ser
                                    50

Gly Ile Tyr Phe Val Tyr Ser Gly Val Val
                                    60

Phe Ser Gly Lys Ala Tyr Ser Pro Lys Ala
                                    70

Thr Ser Ser Pro Leu Tyr Leu Ala His Glu
                                    80

Val Gly Leu Phe Ser Ser Gly Tyr Pro Phe
                                    90

His Val Pro Leu Leu Ser Ser Gly Lys Met
                                    100

Val Tyr Pro Gly Leu Gln Glu Pro Trp Leu
                                    110

His Ser Met Tyr His Gly Ala Ala Phe Gly
                                    120

Leu Thr Gln Gly Asn Gln Leu Ser Thr His
                                    130

Thr Asn Gly Ile Pro His Leu Val Leu Ser
                                    140

Pro Ser Thr Val Phe Phe Gly Ala Phe Ala
                                    150

Leu.
```

**4.** A process for producing a polypeptide according to claim 1, which comprises the steps of modifying a lymphotoxin cDNA from Escherichia coli HB101/pLT (FERM BP-1226) containing pLT13, prepared from lymphotoxin-producing human T cell hybridoma clone AC-5-8, introducing the resulting cDNA into a phenotypic expression vector, transforming an E. coli strain with said vector, cultivating the transformant to cause gene expression, and isolating the polypeptide from the culture followed by purification.

**Patentansprüche**

**1.** Homogenes physiologisch aktives rekombinantes humanes Lymphotoxinpolypeptid, das (a) im wesentlichen frei von Verunreinigungen ist, (b) als N-terminale Aminosäuresequenz Met-His-Leu-Ala-Ser-Asn-Leu-Lys-Pro-Ala-Ala-His-Leu-Ile-Gly-Asp-Pro-Ser-Lys-Gly-Asn- hat und (c) die folgende Aminosäuresequenz hat:

```
Met His Leu Ala Ser Asn Leu Lys Pro Ala
1                                    10
Ala His Leu Ile Gly Asp Pro Ser Lys Gly
                                     20
Asn Ser Leu Leu Trp Arg Ala Asn Thr Asp
                                     30
Arg Ala Phe Leu Gly Asn Gly Phe Ser Leu
                                     40
Ser Asn Asn Ser Leu Leu Val Pro Thr Ser
                                     50
Gly Ile Tyr Phe Val Tyr Ser Gly Val Val
                                     60
Phe Ser Gly Lys Ala Tyr Ser Pro Lys Ala
                                     70
Thr Ser Ser Pro Leu Tyr Leu Ala His Glu
                                     80
Val Gly Leu Phe Ser Ser Gly Tyr Pro Phe
                                     90
His Val Pro Leu Leu Ser Ser Gly Lys Met

                                     100
Val Tyr Pro Gly Leu Gln Glu Pro Trp Leu
                                     110
His Ser Met Tyr His Gly Ala Ala Phe Gly
                                     120
Leu Thr Gln Gly Asn Gln Leu Ser Thr His
                                     130
Thr Asn Gly Ile Pro His Leu Val Leu Ser
                                     140
Pro Ser Thr Val Phe Phe Gly Ala Phe Ala
                                     150
Leu.
```

2. DNA, kodierend für ein physiologisch aktives Polypeptid mit der Aminosäuresequenz:

Met His Leu Ala Ser Asn Leu Lys Pro Ala
1 10
Ala His Leu Ile Gly Asp Pro Ser Lys Gly
20
Asn Ser Leu Leu Trp Arg Ala Asn Thr Asp
30
Arg Ala Phe Leu Gly Asn Gly Phe Ser Leu
40
Ser Asn Asn Ser Leu Leu Val Pro Thr Ser
50
Gly Ile Tyr Phe Val Tyr Ser Gly Val Val
60
Phe Ser Gly Lys Ala Tyr Ser Pro Lys Ala
70
Thr Ser Ser Pro Leu Tyr Leu Ala His Glu
80
Val Gly Leu Phe Ser Ser Gly Tyr Pro Phe
90
His Val Pro Leu Leu Ser Ser Gly Lys Met
100
Val Tyr Pro Gly Leu Gln Glu Pro Trp Leu
110
His Ser Met Tyr His Gly Ala Ala Phe Gly
120
Leu Thr Gln Gly Asn Gln Leu Ser Thr His
130
Thr Asn Gly Ile Pro His Leu Val Leu Ser
140
Pro Ser Thr Val Phe Phe Gly Ala Phe Ala
150
Leu.

3. Antitumorzusammensetzung, die als Wirkstoff eine für die Tumorbehandlung wirksame Menge eines Polypeptids mit der Aminsäuresequenz enthält:

Met His Leu Ala Ser Asn Leu Lys Pro Ala
1 10
Ala His Leu Ile Gly Asp Pro Ser Lys Gly
20
Asn Ser Leu Leu Trp Arg Ala Asn Thr Asp
30
Arg Ala Phe Leu Gly Asn Gly Phe Ser Leu
40
Ser Asn Asn Ser Leu Leu Val Pro Thr Ser
50
Gly Ile Tyr Phe Val Tyr Ser Gly Val Val
60
Phe Ser Gly Lys Ala Tyr Ser Pro Lys Ala
70
Thr Ser Ser Pro Leu Tyr Leu Ala His Glu
80
Val Gly Leu Phe Ser Ser Gly Tyr Pro Phe
90

His Val Pro Leu Leu Ser Ser Gly Lys Met
100
Val Tyr Pro Gly Leu Gln Glu Pro Trp Leu
110
His Ser Met Tyr His Gly Ala Ala Phe Gly
120
Leu Thr Gln Gly Asn Gln Leu Ser Thr His
130
Thr Asn Gly Ile Pro His Leu Val Leu Ser
140
Pro Ser Thr Val Phe Phe Gly Ala Phe Ala
150
Leu.

4. Verfahren zur Herstellung eines Polypeptids nach Anspruch 1, das die Schritte umfaßt: Modifizieren einer Lymphotoxin-cDNA aus Escherichia coli HB101/pLT (FERM BP-1226) enthaltend pLT13, hergestellt aus Lymphotoxin-produzierendem humanem T-Zell-Hybridoma-Klon A-C5-8, Einführen der erhaltenen cDNA in einen phenotypischen Expressionsvektor, Transformieren eines E. coli-Stammes mit diesem Vektor, Kutivieren der Transformante unter Bewirkung der Genexpression und Isolieren des Polypeptids aus der Kultur und anschließende Reinigung.

## Revendications

**1.** Polypeptide de lymphotoxine humaine recombinante physiologiquement actif homogène qui (a) est sensiblement exempt d'impuretés, (b) a une séquence d'acides aminés N-terminale Met-His-Leu-Ala-Ser-Asn-Leu-Lys-Pro-Ala-Ala-His-Leu-Ile-Gly-Asp-Pro-Ser-Lys-Gly-Asn et (c) a la séquence d'acides aminés suivante :

```
Met His Leu Ala Ser Asn Leu Lys Pro Ala
1                                   10

Ala His Leu Ile Gly Asp Pro Ser Lys Gly
                                    20

Asn Ser Leu Leu Trp Arg Ala Asn Thr Asp
                                    30

Arg Ala Phe Leu Gly Asn Gly Phe Ser Leu
                                    40

Ser Asn Asn Ser Leu Leu Val Pro Thr Ser
                                    50

Gly Ile Tyr Phe Val Tyr Ser Gly Val Val
                                    60

Phe Ser Gly Lys Ala Tyr Ser Pro Lys Ala
                                    70

Thr Ser Ser Pro Leu Tyr Leu Ala His Glu
                                    80

Val Gly Leu Phe Ser Ser Gly Tyr Pro Phe
                                    90

His Val Pro Leu Leu Ser Ser Gly Lys Met
                                    100

Val Tyr Pro Gly Leu Gln Glu Pro Trp Leu
                                    110

His Ser Met Tyr His Gly Ala Ala Phe Gly
                                    120

Leu Thr Gln Gly Asn Gln Leu Ser Thr His
                                    130
```

Thr Asn Gly Ile Pro His Leu Val Leu Ser
140

Pro Ser Thr Val Phe Phe Gly Ala Phe Ala
150

Leu.

2. ADN codant un polypeptide physiologiquement actif ayant la séquence d'acides aminés :

Met His Leu Ala Ser Asn Leu Lys Pro Ala
1 10

Ala His Leu Ile Gly Asp Pro Ser Lys Gly
20

Asn Ser Leu Leu Trp Arg Ala Asn Thr Asp
30

Arg Ala Phe Leu Gly Asn Gly Phe Ser Leu
40

Ser Asn Asn Ser Leu Leu Val Pro Thr Ser
50

Gly Ile Tyr Phe Val Tyr Ser Gly Val Val
60

Phe Ser Gly Lys Ala Tyr Ser Pro Lys Ala
70

Thr Ser Ser Pro Leu Tyr Leu Ala His Glu
80

Val Gly Leu Phe Ser Ser Gly Tyr Pro Phe
90

His Val Pro Leu Leu Ser Ser Gly Lys Met
100

Val Tyr Pro Gly Leu Gln Glu Pro Trp Leu
110

His Ser Met Tyr His Gly Ala Ala Phe Gly
120

Leu Thr Gln Gly Asn Gln Leu Ser Thr His
130

Thr Asn Gly Ile Pro His Leu Val Leu Ser
140

Pro Ser Thr Val Phe Phe Gly Ala Phe Ala
150

Leu.

3. Composition antitumorale qui comprend, en tant qu'ingrédient actif, une quantité efficace pour traiter les tumeurs d'un polypeptide ayant la séquence d'acides aminés :

Met His Leu Ala Ser Asn Leu Lys Pro Ala
1 10

Ala His Leu Ile Gly Asp Pro Ser Lys Gly
20

Asn Ser Leu Leu Trp Arg Ala Asn Thr Asp
30

Arg Ala Phe Leu Gly Asn Gly Phe Ser Leu
40

Ser Asn Asn Ser Leu Leu Val Pro Thr Ser
50

Gly Ile Tyr Phe Val Tyr Ser Gly Val Val
60

Phe Ser Gly Lys Ala Tyr Ser Pro Lys Ala
70

Thr Ser Ser Pro Leu Tyr Leu Ala His Glu
80

Val Gly Leu Phe Ser Ser Gly Tyr Pro Phe
90

His Val Pro Leu Leu Ser Ser Gly Lys Met
100

Val Tyr Pro Gly Leu Gln Glu Pro Trp Leu
110

His Ser Met Tyr His Gly Ala Ala Phe Gly
120

Leu Thr Gln Gly Asn Gln Leu Ser Thr His
130

Thr Asn Gly Ile Pro His Leu Val Leu Ser
140

Pro Ser Thr Val Phe Phe Gly Ala Phe Ala
150

Leu.

4. Procédé de production d'un polypepide selon la revendication 1, qui comprend les étapes de modification d'un ADNc de lymphotoxine provenant de Escherichia coli HB101/pLT (FERM BP-1226) contenant pLT 13, préparé à partir du clone d'hybridome de cellules T humaines producteur de lymphotoxine AC-5-8, d'introduction de l'ADNc résultant dans un vecteur d'expression phénotypique, de transformation d'une souche de E. coli avec ledit vecteur, de culture du transformant pour provoquer l'expression génique et d'isolement du polypeptide à partir de la culture puis de purification.

Fig. 1

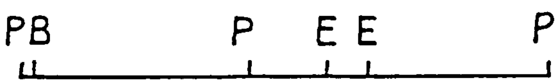


Fig. 2

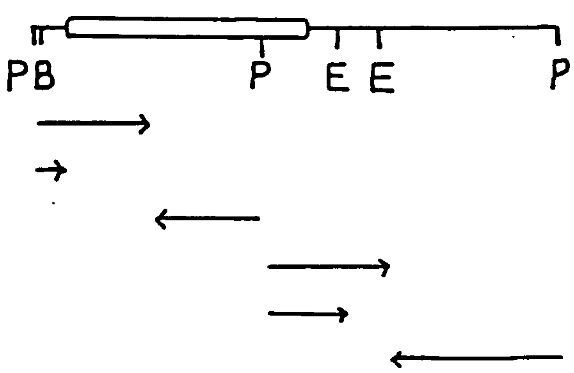

Fig. 3

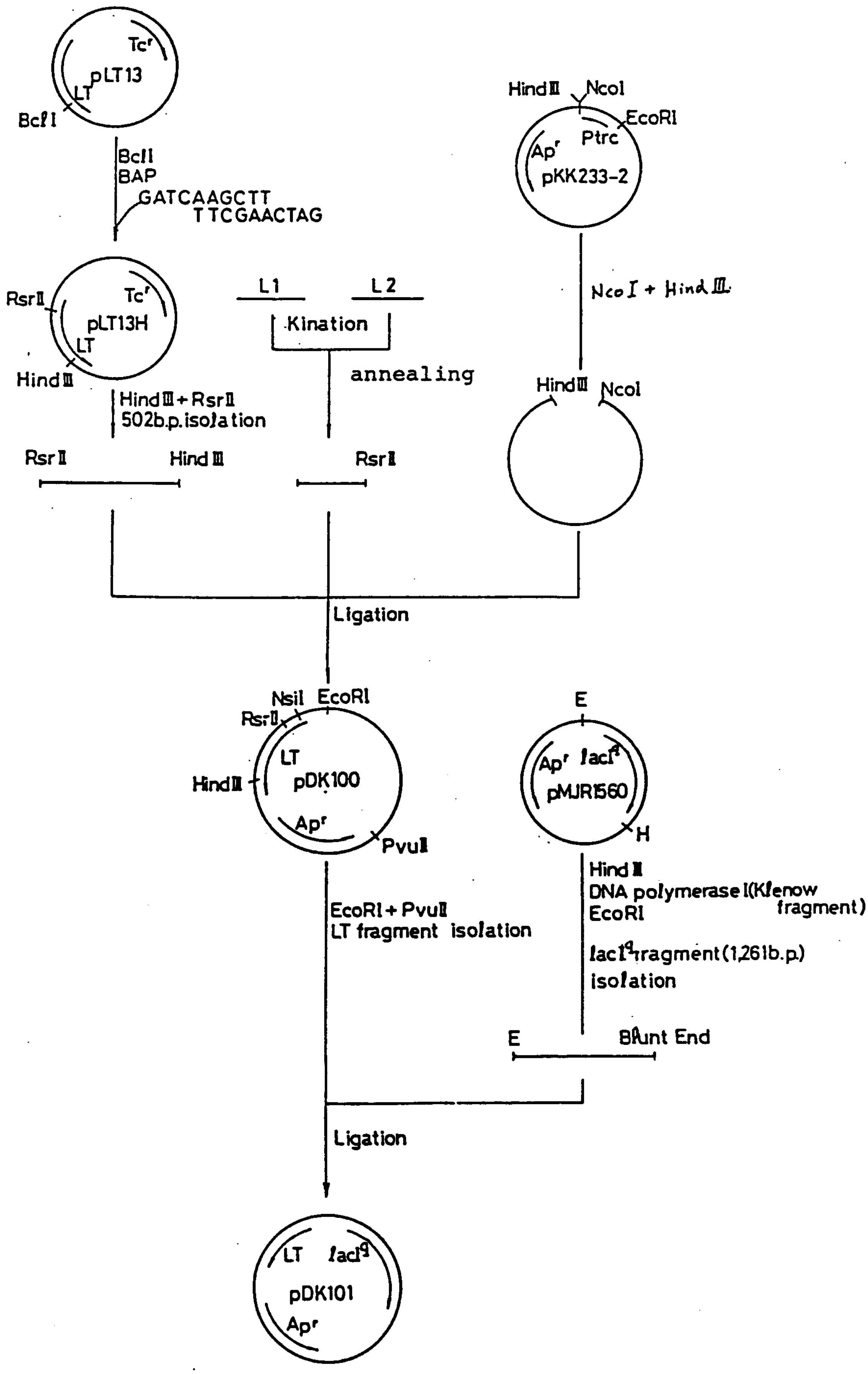
Tc^r
pLT13
LT
BclI
BclI
BAP
GATCAAGCTT
TTCGAACTAG
RsrII
Tc^r
pLT13H
LT
HindIII
HindIII + RsrII
502b.p. isolation
L1
L2
Kination
annealing
HindIII
NcoI
EcoRI
Ptrc
Ap^r
pKK233-2
NcoI + HindIII
HindIII
NcoI
RsrII
HindIII
RsrII
Ligation
NsiI
EcoRI
RsrII
LT
HindIII
pDK100
Ap^r
PvuII
E
Ap^r
lacI^q
pMJR1560
H
HindII
DNA polymerase I (Klenow fragment)
EcoRI
lacI^q fragment (1,261b.p.)
isolation
EcoRI + PvuII
LT fragment isolation
E
Blunt End
Ligation
LT
lacI^q
pDK101
Ap^r

Fig. 4

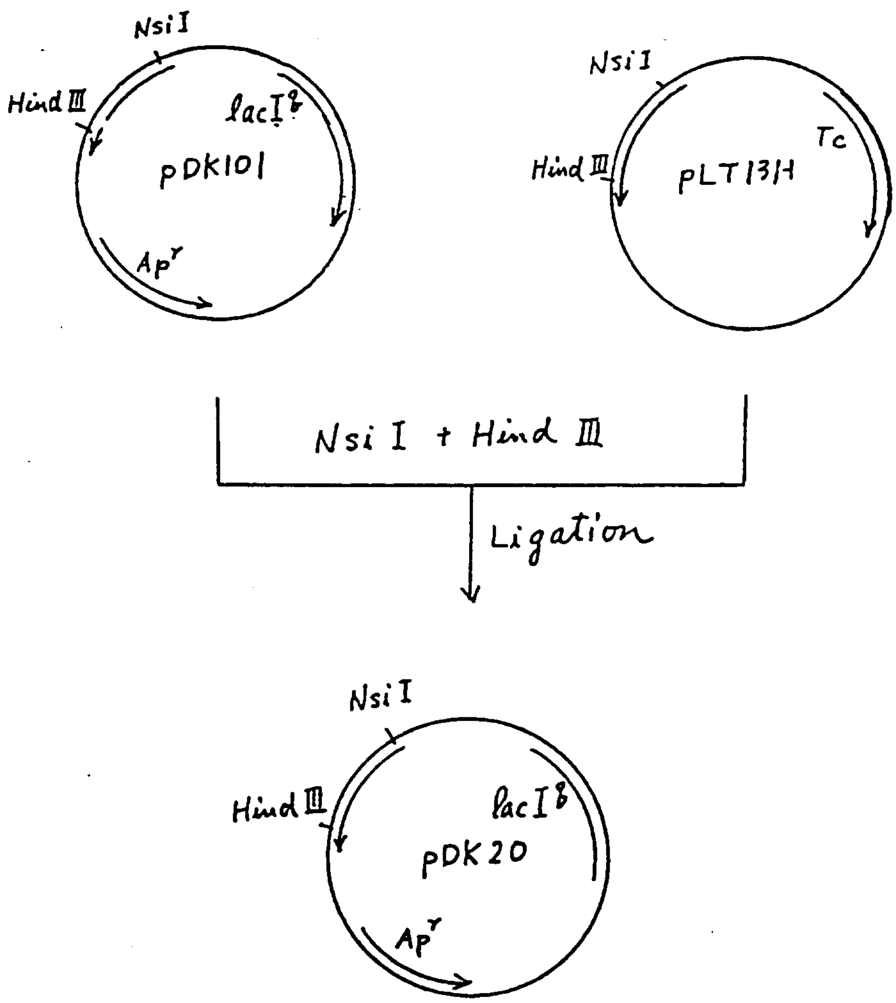
NsiI
HindIII
lacI^q
pDK101
Ap^r
NsiI
HindIII
Tc
pLT13H
NsiI + HindIII
Ligation
NsiI
HindIII
lacI^q
pDK20
Ap^r